# FASCICULE DE BREVET EUROPEEN

(11) **EP 0 974 598 B1**
(45) Date de publication et mention de la délivrance du brevet: **24.08.2011**
(21) Numéro de dépôt: 99401706.9
(22) Date de dépôt: 08.07.1999
(51) Int. Cl.: C07H 17/08, A61K 31/70

(54) **Nouveaux dérivés de l'érythromycine, leur procédé de préparation et leur application comme médicaments**
Neue Erythromycin-Derivate, Verfahren zu deren Herstellung sowie deren Verwendung als Arzneimittel
New erythromycin derivatives, process for making them and their use as medicines

(30) Priorité: 09.07.1998 FR 9808795; 26.04.1999 FR 9905245
(43) Date de publication de la demande: 26.01.2000
(73) Titulaire: Aventis Pharma S.A., 92160 Antony (FR)
(72) Inventeur: Denis, Alexis, 75011 Paris (FR); Gigliotti, Giuseppe, 75011 Paris (FR)
(74) Mandataire: Sartorius, Jérome

(56) Documents cités:
- EP-A- 0 487 411
- WO-A-97/17356
- WO-A-98/09978
- WO-A-99/16779
- FR-A- 2 745 290

## Description

La présente invention concerne de nouveaux derivés de l'érythromycine, leur procédé de préparation et leur application comme médicaments. La demande FR 2 745 290 divulgue des dérivés de l'érythromycine hydroxylis en position 3 et dont l'atome d'azote n'est pas au moins partiellement déméthylé.

L'invention a pour objet les composés de formule (I) 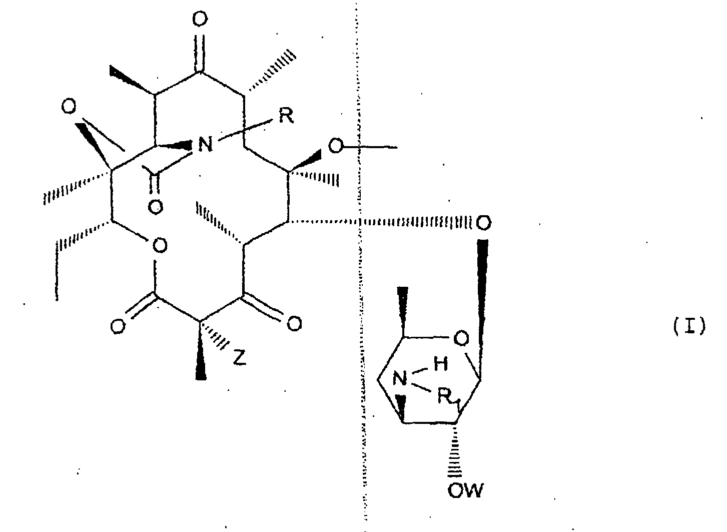 dans laquelle représente un radical

(CH₂)ₘOₙ(X)YAr

dans lequel m représente le nombre 0 ou 1,
n représente le nombre 0 ou 1,
X représente un radical (NH)ₐ, CH₂ ou SO₂ avec a représentant le nombre 0 ou 1,
Y représente un radical (CH₂)_{b}-(CH=CH)_{c}-(CH₂)_{d} avec c = O ou 1, b + c + d ≤ 8,
Z représente un atome d'hydrogène ou d'halogène,
Ar représente un radical aryle ou hétéroaryle éventuellement substitués,
R₁ représente un atome d'hydrogène ou un radical méthyle,
W représente un atome d'hydrogène ou un radical acyle, ainsi que leurs sels d'addition avec les acides.

Parmi les sels d'addition avec les acides, on peut citer les sels formés avec les acides acétique, propionique, trifluoroacétique, maléique, tartrique, méthanesulfonique, benzènesulfonique, p-toluènesulfonique, et spécialement les acides stéarique, éthylsuccinique ou laurylsulfonique.

Le radical aryle peut-être un radical phényle ou naphtyle.

Le radical hétérocylique substitué ou non peut-être le radical thiényle, furyle, pyrrolyle, thiazolyle, oxazolyle, imidazolyle, par exemple le radical 4-(3-pyridinyl) 1H-imidazolyle, thiadiazolyle, pyrazolyle ou isopyrazolyle, un radical pyridyle, pyrimidyle, pyridazinyle ou pyrazinyle, ou encore un radical indolyle, benzofurannyle, benzothiazyle ou quinoléinyle.

Ces radicaux aryles peuvent comporter un ou plusieurs groupements choisis dans le groupe constitué par les radicaux hydroxyle, les atomes d'halogène, les radicaux NO₂, les radicaux C=N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 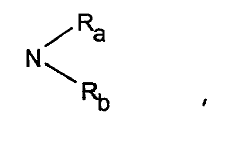 Rₐ et R_{b} identiques ou différents, représentant
un atome d'hydrogène ou un radical alkyle renfermant jusqu'à 12 atomes de carbone, le radical 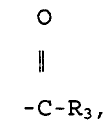 R₃ représentant un radical alkyle renfermant jusqu'à 12 atomes de carbone, ou un radical aryle ou hétéroaryle éventuellement substitué, les radicaux aryle, O-aryle ou S-aryle carboxyliques ou aryle, O-aryle ou S-aryle hétérocycliques à 5 ou 6 chaînons comportant un ou plusieurs hétéroatomes, éventuellement substitués par un ou plusieurs des substituants mentionnés ci-dessous.

Comme hétérocycle préféré, on peut citer entre autres 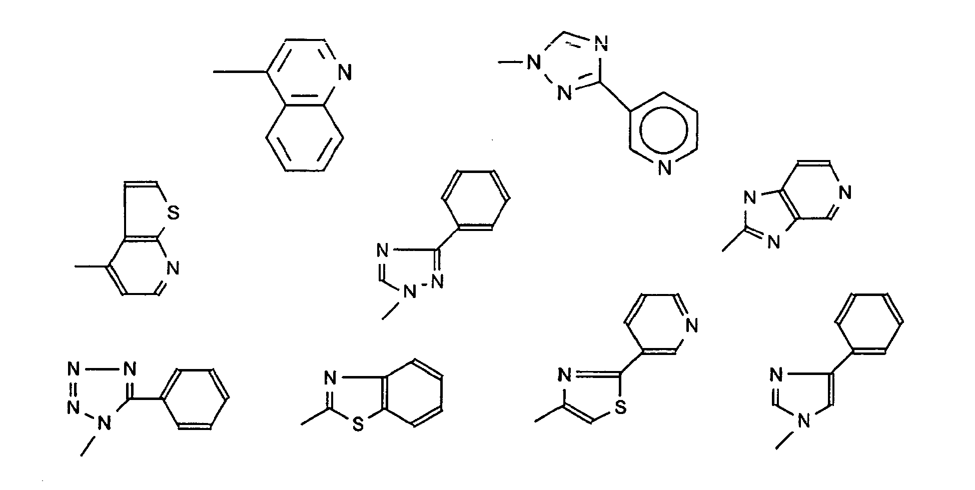 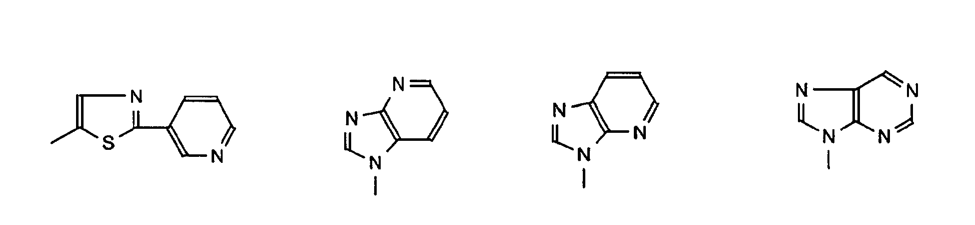 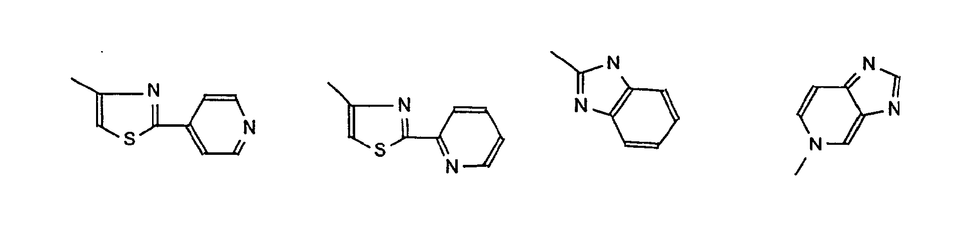 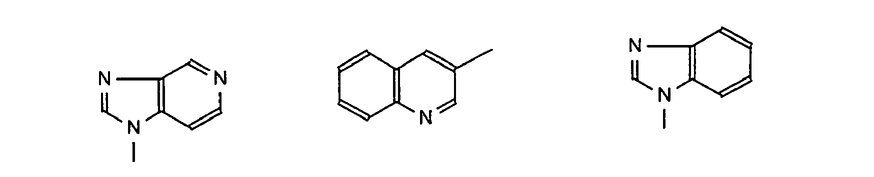 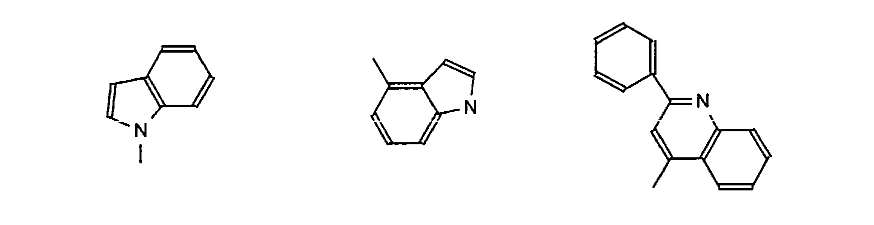 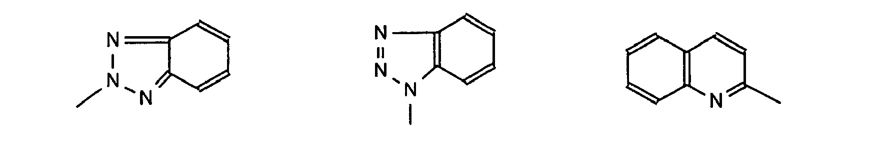 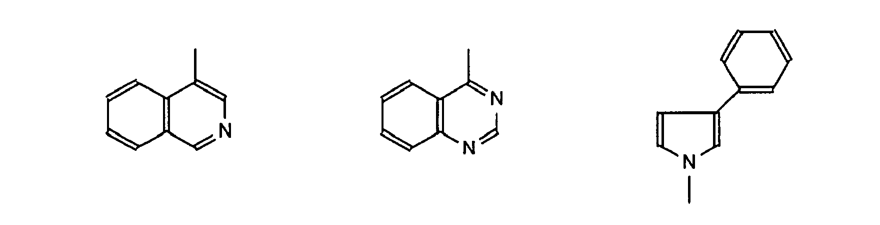 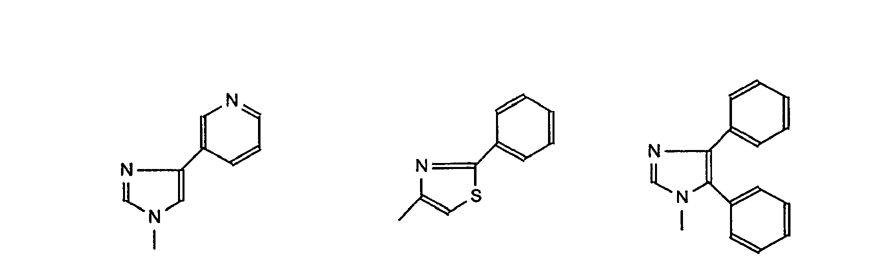 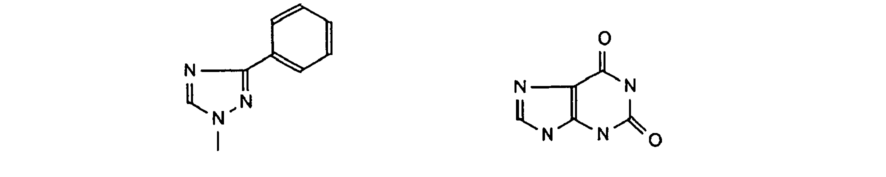 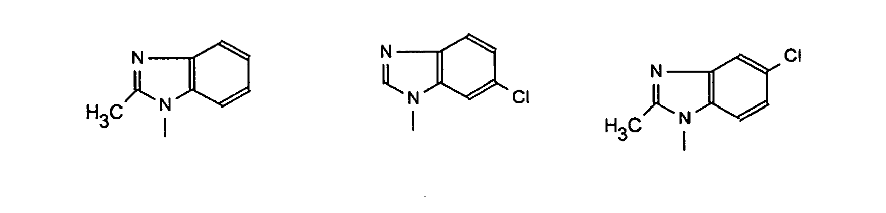 et les radicaux hétérocycliques envisagés dans les demandes de brevets européens 487411, 596802, 676409 et 680967. Ces radicaux hétérocycliques préférés peuvent être substitués par un ou plusieurs groupements fonctionnels.

Halogène représente de préférence un atome de fluor, de chlore ou de brome.

L'invention a notamment pour objet les composés de formule (I) dans lesquels Z représente un atome d'hydrogène, ceux dans lesquels W représente un atome d'hydrogène, ceux dans lesquels R₁ représente un radical méthyle, ceux dans lesquels X représente un radical CH₂.

L'invention a plus spécialement pour objet les composés dans lesquels R représente un radical (CH₂)₃ Ar, (CH₂)₄Ar ou (CH₂)₅ Ar, Ar conservant sa signification précédente.

L'invention a tout spécialement pour objet les composés de formule (I) dans lesquels Ar représente un radical :

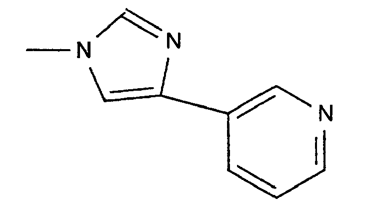

L'invention a tout particulièrement pour objet les composés de formule (I) dont la préparation détaillée est donnée ci-après dans la partie expérimentale.

Les produits de formule générale (I) possèdent une très bonne activité antibiotique sur les bactéries gram ^{⊕} telles que les staphylocoques, les streptocoques, les pneumocoques. Les composés de l'invention peuvent donc être utilisés comme médicaments dans le traitement des infections à germe sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Moraxella catarrhalis, Rickettsies, Mycoplasma pneumoniae, Chlamydia, Legionella, Ureaplasma, Toxoplasma ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, le produit de l'exemple 1 et le produit de l'exemple 2 ainsi que leurs sels pharmaceutiquement acceptables.

L'invention a également pour objet les compositions pharmaceutiques renfermant comme principe actif au moins un des médicaments défini ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut-être, par exemple, comprise entre 50 mg et 1000 mg par jour par voie orale, par exemple de 300 à 900 mg chez l'adulte pour le produit de l'exemple 1.

L'invention a également pour objet un procédé de préparation des composés de formule (I) caractérisé en ce que l'on soumet le composé.de formule (II), 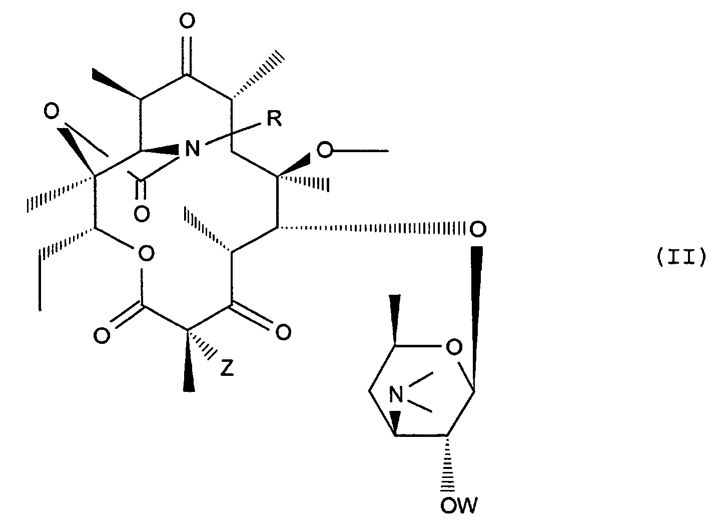 dans laquelle R, Z et W conservent leur signification précédente à l'action d'un agent de déméthylation pour obtenir un mélange de composés de formule (I) déméthylé et didéméthylé correspondants, que l'on sépare pour obtenir le composé de formule I recherché.

Les composés déméthylé et didéméthylé sont séparés selon les procédés classiques par exemple par chromatographie. Les composés de formule (II) utilisés comme produits de départ sont décrits notamment dans les brevets européens 0487411, 596802, 606024, 614905 et 680967.

Comme agent de déméthylation on peut utiliser l'azodicarboxylate de diéthyle ;ou encore l'iode en présence d'acétate de sodium.

### EXEMPLE 1 : N-déméthyl-11,12-dideoxy-3-de[(2,6-dideoxy-3-C-méthyl-3-O-méthyl-alpha-L-ribohexopyranosyl)-oxy]-6-O-méthyl-3-oxo-12,11-[oxycarbonyl[[4-[4-(3-pyridinyl)-1H-imidazol-1-yl]butyl]imino]]-érythromycine.

On introduit 10 g de 11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl((4-(4-(3-pyridinyl)-1H-imidazol-1-yl) butyl)imino))-érythromycine dans 150 cm³ d'acétone. On agite le mélange réactionnel jusqu'à dissolution et introduit 3,83 cm³ d'azodicarboxylate de diéthyle. On maintient sous agitation pendant 3 heures, amène à sec et obtient 14,48 g de produit que l'on chromatographie sur silice en éluant avec le mélange chlorure de méthylène/méthanol/ ammoniaque (90-10-1). On obtient 3,16 g de produit recherché brut. Après recristallisation dans l'héptane, on obtient le produit recherché.
titre HPLC 86%

| | | |
|---|---|---|
| Microanalyse | C:62,3% | théorie 63,22% |
| | H: 8,1% | théorie 7,96% |
| | N: 8,8% | théorie 8,78% |

### EXEMPLE 2 : N-déméthyl-11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl(2-(3-(4-quinoleinyl)-propyl)-hydrazono))-érythromycine

En opérant comme précedemment à partir de la 11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl-(2-(3-(4-quinoleinyl)propyl)hydrazono))-érythromycine, on a obtenu le produit recherché

### EXEMPLE 3 : N-déméthyl-11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl((4-phénylbutyl)imino))-érythromycine.

En opérant comme à l'exemple 1 à partir de la 11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl-((4-phénylbutyl)imino))-érythromycine, on a obtenu le produit recherché.
rf = 0,14 (éther isopropylique-méthanol-triéthylamine 80-10-10).

### EXEMPLE 4 : N-didéméthyl-11,12-dideoxy-3-de[(2,6-dideoxy-3-C-méthyl-3-O-méthyl-alpha-L-ribohexopyranosyl)oxy]-6-O-méthyl-3-oxo-12,11-[oxycarbonyl[[4-[4-(3-pyridinyl)-1H-imidazol-1-yl]butyl]imino]]-érythromycine.

On ajoute à 20 ~ 25°C, 7,7 cm³ de diethylazodi-carboxylate dans une solution renfermant 20 g de 11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl-((4-(4-(3-pyridinyl)-1H-imidazol-1-yl) butyl)-imino))-érythromycine dans 300 cm³ d'acétone. On agite 4 heures à 20 ≈ 25°C. On amène à sec. On obtient 28,70 g de produit que l'on chromatographie sur silice en éluant avec le mélange chlorure de méthylène-méthanol-ammoniaque 90-10-01. On recueille la première fraction que l'on amène à sec et obtient 14 g de produit que l'on introduit dans un mélange de 140 cm³ de méthanol et 70 cm³ d'une solution d'acide chlorhydrique 2N. On agite pendant 24 heures. On amène le pH à 7 par addition d'ammoniaque à 20%. On extrait au chlorure de méthylène. On réextrait la phase aqueuse avec du chlorure de méthylène. On sèche les phases organiques avec du sulfate de sodium. On filtre, amène à sec le filtrat. On obtient 14,4 g du produit que l'on chromatographie sur silice. On élue avec un mélange chlorure de méthylène-méthanol-ammoniaque 90-10-01. On recueille le produit attendu brut, amené à sec par distillation pression réduite. On obtient 5,4 g du produit recherché brut que l'on empâte dans l'heptane. On agite une heure à 20 ≈ 25°C le produit obtenu, le lave, l'essore et le sèche. On Obtient 4,5 g de produit recherché.
Analyse C 41 H 61 N 5 O 10

| | Calculé | Trouvé |
|---|---|---|
| C | 62,82 | 62,5 |
| H | 7,84 | 7,9 |
| N | 8,93 | 8,9 |

### EXEMPLE DE COMPOSITIONS PHARMACEUTIQUES

On a préparé des compositions renfermant :

| | |
|---|---|
| **Produit de l'exemple 1** | 300 mg |
| Excipient q.s.p | 1 g |

Détail de l'excipient : amidon, talc,
stéarate de magnésium

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus avec le produit de l'exemple 1 et de l'exemple 2 (lecture après 24 heures)

| | | | **Ex. 1** | **Ex. 2** |
|---|---|---|---|---|
| S.aureus | 011UC4 | ery S | 0.300 | 0.080 |
| S.aureus | 011UC4 + sérum 50% | ery S | 0.040 | 0.150 |
| S.aureus | o11B18c | oxa S ery R | | |
| S.aureus | 011GR12c | oxa S ery R | | |
| S.aureus | 011GO25i | oxa S ery R | 5.000 | 0.600 |
| S. epidermidis | 012GO11i | oxa S ery R | 0.150 | 0.300 |
| S.aureus | 011CB20c | oxa R ery R | | |
| S. epidermidis | 012GO40c | oxa R ery R | | |
| S. pyogenes | 02A1UC1 | ery S | 0.800 | 0.040 |
| S. agalactiae | 02B1HT1 | ery S | <=0.02 | 0.020 |
| E. faecalis | 02D2UC1 | ery S | 0.080 | 0.040 |
| E. faecium | 02D3HT1 | ery S | 0.080 | 0.040 |
| Streptococcus gr.G | 02GOGR5 | ery S | 0.080 | 0.040 |
| S. mitis | 02MitCB1 | ery S | 0.080 | <=0.01 |
| S. pyogenes | 02A1SJc | ery R | | |
| S. agalactiae | 02B1SJ1c | ery R | 0.600 | 0.150 |
| E. faecalis | 02D2DU15c | ery R | >40 | >20 |
| Streptococcus gr.G | 02GOgr4c | ery R | | |
| S. sanguis | 02SgGr10i | ery R | 0.150 | 0.150 |
| S. mitis | 02MitGR16i | ery R | 0.080 | 0.080 |
| S. pneumoniae | 032UC1 | ery S | <=0.02 | <=0.01 |
| S. pneumoniae | 030GR20 | ery S | <=0.02 | <=0.01 |
| S. pneumoniae | 030SJ5i | ery R | 0.080 | 0.080 |
| S. pneumoniae | 030CR18c | ery R | 10.000 | 2.500 |
| S. pneumoniae | 030PW23c | ery R | 0.150 | 0.080 |
| S. pneumoniae | 030RO1i | ery R | 0.150 | 0.300 |
| S. pneumoniae | 030SJ1c | ery R | 0.080 | 0.150 |

## Revendications

1. Les composés de formule (I) 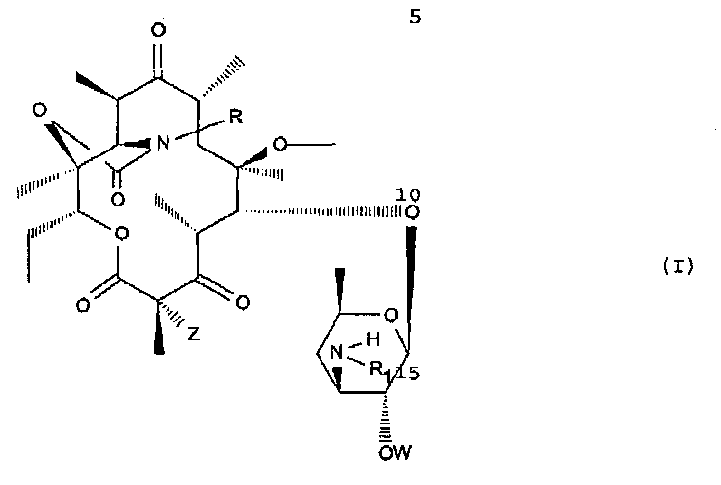 dans laquelle R représente un radical
(CH₂)ₘOₙ(X) YAr
dans lequel m représente le nombre 0 ou 1,
n représente le nombre 0 ou 1,
X représente un radical (NH)ₐ, CH₂ ou SO₂ avec a représentant le nombre 0 ou 1 ,
Y représente un radical (CX₂)_{b}-(CH=CH)_{c}-(CH₂)_{d} avec c = O ou 1, b + c + d ≤ 8,
z représente un atome d'hydrogène ou d'halogène,
Ar représente un radical aryle ou hétéroaryle éventuellement substitué, un ou plusieurs groupements choisis dans le groupe constitué par les radicaux hydroxyle, les atomes d'halogène, les radicaux NO₂, les radicaux C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 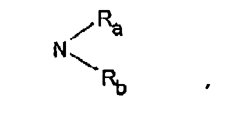 Rₐ et R_{b} identiques ou différents, représentant un atome d'hydrogène ou un radical alkyle renfermant jusqu'à 12 atomes de carbone, le radical 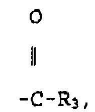 R₃ représentant un radical alkyle renfermant jusqu'à 12 atomes de carbone, ou un radical aryle ou hétéroaryle
R₁ représente un atome d'hydrogène ou un radical méthyle W représente un atome d'hydrogène ou un radical acyle, ainsi que leurs sels d'addition avec les acides

2. Les composés de formule (I) définis à la revendication 1 dans lesquels Z représente un atome d'hydrogène.

3. Les composés de formule (I) définis à la revendication 1 ou 2, dans lesquels W représente un atome d'hydrogène.

4. Les composés de formule I définis à la revendication 1, 2 ou 3 dans lesquels R₁ représente un radical méthyle.

5. Les composés de formule (I) définis à la revendication 1, 2, 3 ou 4 dans lesquels X représente un radical CH₂.

6. Les composés de formule (I) définis à l'une quelconque des revendications 1 à 5 dans lesquels R représente un radical (CH₂)₃ Ar, (CH₂)₄Ar ou (CH₂)₅ Ar, Ar conservant sa signification indiquée à la revendication 1.

7. Les composés de formule (I) définis à l'une quelconque des revendications 1 à 6 dans lesquels Ar représente un radical

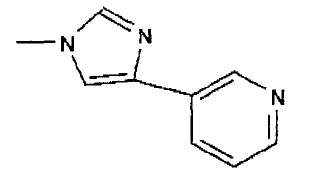

8. Les composés de formule (I) définis à la revendication 1 dont les noms suivent :
- N-déméthyl-11,12-dideoxy-3-de[(2,6-dideoxy-3-C-méthyl-3-O-méthyl-alpha-L-ribo-hexopyranosyl)oxy]-6-0-méthyl-3-oxo-12,11-[oxycarbonyl[[4-[4-(3-pyridinyl)-1H-imidazol-1-yl]butyl]imino]]-érythromycine,
- N-déméthyl-11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl(2-(3-(4-quinoleinyl)propyl)hydrazono))-érythromycine,
- N-didéméthyl-11,12-dideoxy-3-de[(2,6-dideoxy-3-C-méthyl-3-O-méthyl-alpha-L-ribohexopyranosyl)oxy]-6-O-méthyl-3-oxo-12,11-[oxycarbonyl[[4-[4-(3-pyridinyl)-1H-imidazol-1-yl]butyl]imino]]-érythromycine.

9. A titre de médicaments, les composés de formule (I) définis à l'une quelconque des revendications 1 à 7, ainsi que leurs sels pharmaceutiquement acceptables.

10. A titre de médicaments les composés de la revendication 8 ainsi que leurs sels pharmaceutiquement acceptables.

11. Les compositions pharmaceutiques renferment comme principe actif au moins un médicament selon la revendication 9 ou 10.

12. Procédé de préparation des composés de formule (I) **caractérisé en ce que** l'on soumet le composé de formule (II), 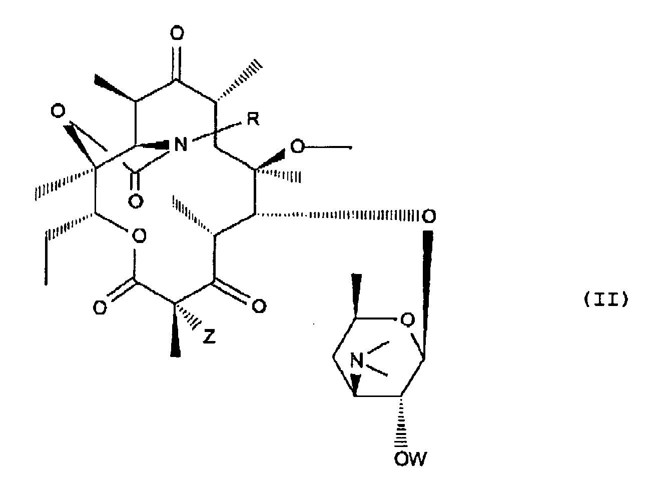 dans laquelle R, Z et W conservent leur signification indiquée à la revendication 1, à l'action d'un agent de déméthylation pour obtenir un mélange de composés déméthylés et didéméthylés de formule (I) correspondant que l'on sépare pour obtenir le composé de formule I recherché.

## Claims

1. Compounds of formula (I) 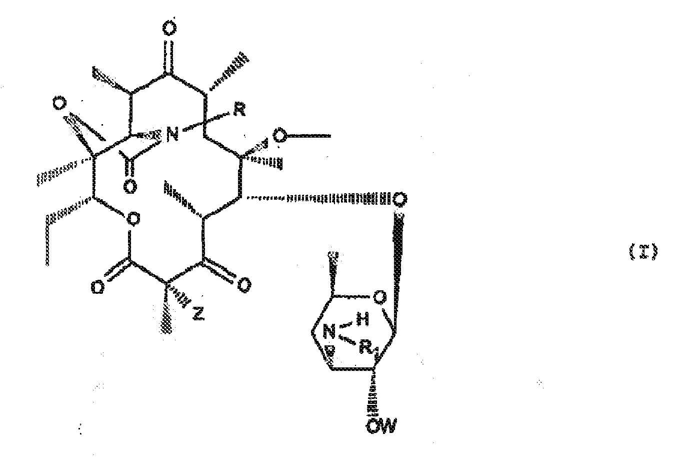 in which R represents the following radical
(CH₂)ₘOₙ(X)YAr
in which m represents the number 0 or 1,
n represents the number 0 or 1,
X represents an (NH)ₐ, CH₂ or SO₂ radical with a representing the number 0 or 1,
Y represents a (CH₂)_{b}-(CH=CH)_{c}-(CH₂)_{d} radial with c = 0 or 1 and b + c + d ≤ 8,
Z represents a hydrogen or halogen atom,
Ar represents an aryl or heteroaryl radical optionally substituted by one or more groups chosen from the group consisting of hydroxyl radicals, halogen atoms, NO₂ radicals, C≡N radicals, alkyl, alkenyl, alkynyl, 0-alkyl, O-alkenyl, O-alkynyle, S-alkyl, S-alkenyl, S-alkynyl, N-alkyl, N-alkenyl and N-alkynyl radicals, including up to 12 carbon atoms optionally substituted by one or more halogen atoms, the 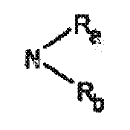 radical, Rₐ and R_{b}, which are identical or different, representing a hydrogen atom or an alkyl radical including up to 12 carbon atoms, the 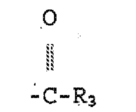 radical, R₃ representing an alkyl radical including up to 12 carbon atoms, an aryl radical and a heteroaryl radical,
R₁ represents a hydrogen atom or a methyl radical,
W represents a hydrogen atom or an.acyl radical,
and their addition salts with acids.

2. Compounds of formula (I) which are defined in Claim 1, in which Z represents a hydrogen atom.

3. Compounds of formula (I) which are defined in Claim 1 or 2, in which W represents a hydrogen atom.

4. Compounds of formula (I) which are defined in Claim 1, 2 or 3, in which R₁ represents a methyl radical.

5. Compounds of formula (I) which are defined in Claim 1, 2, 3 or 4, in which X represents a CH₂ radical.

6. Compounds of formule (I) which are defined in any one of Claims 1 to 5, in which R represents a (CH₂)₃Ar, (CH₂)₄Ar or (CH₂)₅Ar radical, Ar retaining its meaning shown in Claim 1.

7. Compounds of formula (I) which are defined in any one of Claims 1 to 6, in which Ar represents a 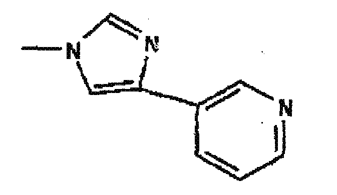 radical.

8. The compounds of formula (I) which are defined in Claim 1, the names of which follow:
- N-demethyl-11,12-dideoxy-3-de[(2,6-dideoxy-3-C-methyl-3-O-methyl-α-L-ribo-hexopyranosyl)oxy]-6-O-methyl-3-oxo-12,11-[oxycarbonyl[[4-[4-(3-pyridinyl)-1H-imidazol-1-yl]butyl]imino]]erythromycin,
- N-demethyl-11,12-dideoxy-3-de((2,6-dideoxy-3-C-methyl-3-O-methyl-α-L-ribo-hexopyranosyl)oxy)-6-O-methyl-3-oxo-12,11-(oxycarbonyl(2-(3-(4-quinolyl)propyl)hydrazono))erythromycin,
- N-didemethyl-11,12-dideoxy-3-de[(2,6-dideoxy-3-C-methyl-3-O-methyl-α-L-ribo-hexopyranosyl)oxy]-6-O-methyl-3-oxo-12,11-[oxycarbonyl[[4-[4-(3-pyridinyl)-1H-imidazol-1-yl]butyl]imino]]erythromycin.

9. As medicaments, the compounds of formula (I) which are defined in any one of Claims 1 to 7 and their pharmaceutically acceptable salts.

10. As medicaments, the compounds of Claim 8 and their pharmaceutically acceptable salts.

11. Pharmaceutical compositions including, as active principle, at least one medicament according to Claim 9 or 10.

12. Process for the preparation of the compounds of formula (I), **characterized in that** the compound of formula (II): 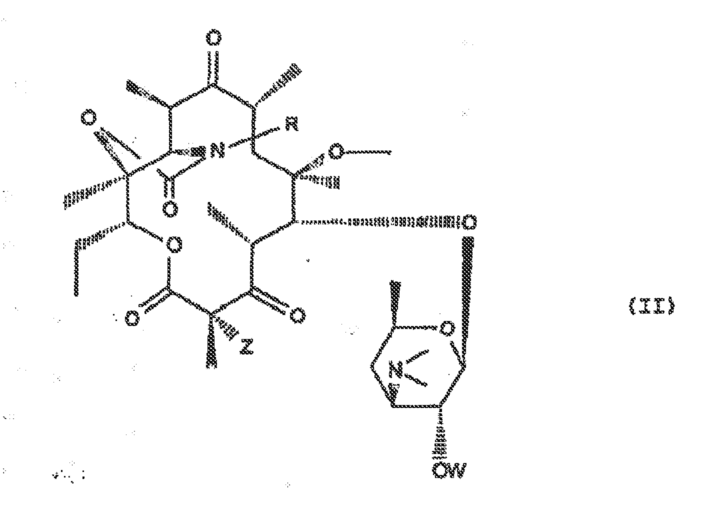 in which R, Z and W retain their meaning shown in Claim 1, is subjected to the action of a demethylating agent in order to obtain a mixture of demethylated and didemethylated compounds of corresponding formula (I) which are separated in order to obtain the desired compound of formula (I).

## Patentansprüche

1. Verbindungen der Formel (I) 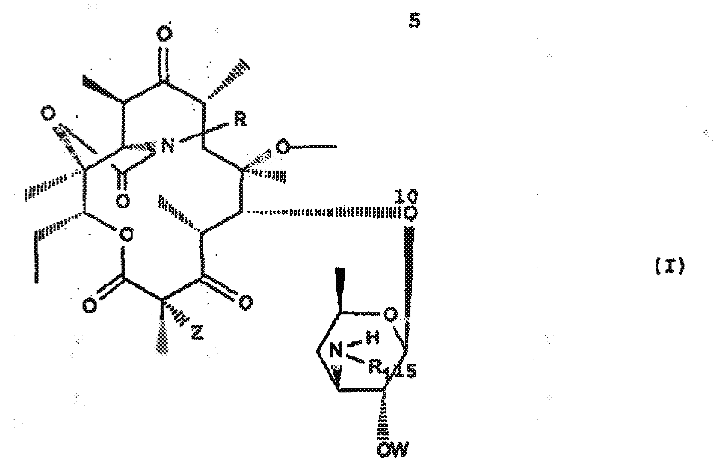 in welcher R einen nachfolgend gezeigten Rest darstellt:
(CH₂)ₘOₙ(X) YAr
in der m für die Zahl 0 order 1 steht,
n für die Zahl 0 oder 1 steht,
X für einen Rest (NH)ₐ, CH₂ oder SO₂ steht, wobei a für die Zahl 0 oder 1 steht,
Y für einen Rest (CH₂)_{b}-(CH=CH)_{c}-(CH₂)_{d} steht, wobei c = 0 oder 1, b + c + d ≤ 8,
Z für ein Wasserstoff- oder Halogenatom steht,
Ar für einen Aryl- oder Heteroarylrest steht, der gegebenenfalls durch eine oder mehrere Gruppen substituiert ist, die aus der Gruppe bestehend aus Hydroxylresten, Halogenatomen, NO₂-Resten, C≡N-Resten, Alkyl-, Alkenyl- oder Alkinyl-, O-Alkyl-, O-Alkenyl- oder O-Alkinyl-, S-Alkyl-, S-Alkenyl oder S-Alkinyl- und N-Alkyl-, N-Alkenyl- oder N-Alkinylresten ausgewählt wird, wobei diese bis zu 12 Kohlenstoffatomen umfassen und gegebenenfalls durch ein oder mehrere Halogenatome substituiert sind, oder dem folgende Radikal 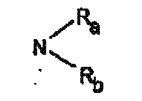 in dem Rₐ und R_{b}, identisch oder verschieden, für ein Wasserstoffatom oder einen bis zu 12 Kohlenstoffatomen umfassenden Alkylrest stehen, den Resten 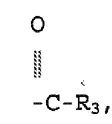 wobei R₃ für einen bis zu 12 Kohlenstoffatomen umfassenden Alkylrest steht, oder einem Aryl- oder Heteroarylrest ausgewählt wird,
R₁ für ein Wasserstoffatom oder einen Methylrest steht, W für ein Wasserstoffatom oder einen Acylrest steht, sowie deren Additionssalze mit Säuren.

2. Verbindungen der Formel (I) wie im Anspruch 1 definiert, in der Z für ein Wasserstoffatom steht.

3. Verbindungen der Formel (I) wie im Anspruch 1 oder 2 definiert, in der W für ein Wasserstoffatom steht.

4. Verbindungen der Formel (I) wie in Anspruch 1, 2 oder 3 definiert, in der R₁ für einen Methylrest steht,

5. Verbindungen der Formel (I) wie in Anspruch 1, 2, 3 oder 4 definiert, in der X für einen CH₂-Rest steht.

6. Verbindungen der Formel (I) wie in einem der Ansprüche 1 bis 5 definiert, in der R für einen Rest (CH₂)₃Ar, (CH₂)₄Ar oder (CH₂)₅ Ar steht, wobei Ar seine in Anspruch 1 angegebene Bedeutung behält.

7. Verbindungen der Formel (I) wie in einem der Ansprüche 1 bis 6 definiert, in der Ar für einen unten folgenden Rest steht

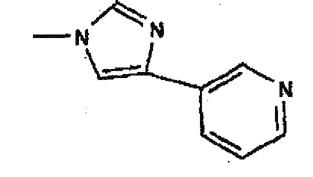

8. Verbindungen der Formel (I) wie in Anspruch 1 definiert, mit den folgenden Bezeichnungen:
- N-Demethyl-11,12-dideoxy-3-de[(2,6-dideoxy-3-C-methyl-3'-O-methyl-α-L-ribohexopyranosyl)oxy]-6-O-methyl-3-oxo-12,11-(oxycarbonyl-[(4-[4-(3-pyridinyl)-1H-imidazol-1-yl]butyl)imino]}erythromycin,
- N-Demethyl-11,12-dideoxy-3-de[(2,6-dideoxy-3-C-methyl-3-O-methyl-α-L-ribohexopyranosyl)oxy]-6-O-methyl-3-oxo-12,11-{oxycarbonyl-[2-(3-(4-chinoleinyl)propyl) hydrazono]}erythromycin,
- N-Didemethyl-11,12-dideoxy-3-de[(2,6-dideoxy-3-C-methyl-3-O-methyl-α-L-ribohexopyranosyl)oxy]-6-O-methyl -3-oxo-12,11-{oxycarbonyl-[(4-[4-(3-pyridinyl)-1H-imidazol-1-yl]butyl)imino]}erythromycin.

9. Verbindungen der Formel (I) wie in einem der Ansprüche 1 bis 7 definiert, sowie deren pharmazeutisch unbedenkliche Salze als Arzneimittel.

10. Verbindungen gemäß Anspruch 8, sowie deren pharmazeutisch unbedenkliche Salze als Arzneimittel.

11. Pharmazeutische Zusammensetzungen, umfassend mindestens ein Arzneimittel nach Anspruch 9 oder 10 als Wirkstoff.

12. Verfahren zur Herstellung der Verbindungen der Formel (I) **dadurch gekennzeichnet, dass** eine Verbindung der folgenden Formel (II) 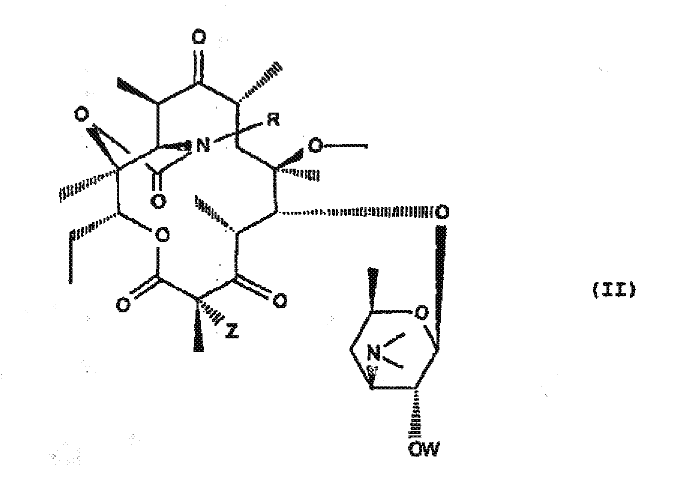 in der R, Z und W ihre in Anspruch 1 angegebenen Bedeutungen behalten, der Wirkung eines demethylierenden Mittels unterzogen wird, um eine Mischung aus korrespondierenden, demethylierten und didemethylierten Verbindungen der Formel (1) zu erhalten, die getrennt werden, um die gesuchte Verbindung der Formel (I) zu erhalten.
